(19) Europäisches Patentamt / European Patent Office / Office européen des brevets

(11) EP 1 625 135 B1

(12) FASCICULE DE BREVET EUROPEEN

(45) Date de publication et mention de la délivrance du brevet:
**08.04.2009 Bulletin 2009/15**

(21) Numéro de dépôt: **04710418.7**

(22) Date de dépôt: **12.02.2004**

(51) Int Cl.:
*C07H 11/00* (2006.01) *C07H 1/06* (2006.01)

(86) Numéro de dépôt international:
**PCT/FR2004/000314**

(87) Numéro de publication internationale:
**WO 2004/078105 (16.09.2004 Gazette 2004/38)**

# (54) COMPOSITION DE FONDAPARINUX SODIQUE DE HAUTE PURETE

FONDAPARINUX- NATRIUM ZUSAMMENSTELLUNG VON HOHER REINHEIT

FONDAPARINUX SODIUM COMPOSITION OF HIGH PURITY

(84) Etats contractants désignés:
**AT BE BG CH CY CZ DE DK EE ES FI FR GB GR HU IE IT LI LU MC NL PT RO SE SI SK TR**
Etats d'extension désignés:
**LT LV**

(30) Priorité: **27.02.2003 US 375268**

(43) Date de publication de la demande:
**15.02.2006 Bulletin 2006/07**

(73) Titulaire: **GLAXO GROUP LIMITED**
**Greenford,**
**Middlesex UB6 0NN (GB)**

(72) Inventeurs:
- **BRANELLEC, Jean-François**
  **F-76130 Mont Saint-Aignan (FR)**
- **MORELLO, Christian**
  **F-34830 Clapiers (FR)**
- **POTIER, Pierre**
  **F-04200 Noyers Sur Jabron (FR)**
- **TROUILLEUX, Patrick**
  **F-04200 Sisteron (FR)**
- **BASTIAANSEN, Petrus Marcus F. M.,**
  **Diosynth B.V.**
  **NL-5340 BH OSS (NL)**
- **CLAASSEN, Henricus, Cornelis, Jozephus**
  **NL-5447 AB Rijkevoort (NL)**

(74) Mandataire: **Sewell, Richard Charles et al**
**GlaxoSmithKline**
**Corporate Intellectual Property**
**CN925.1**
**980 Great West Road**
**Brentford, Middlesex TW8 9GS (GB)**

(56) Documents cités:
**DE-A- 19 543 052** **FR-A- 2 557 139**
**US-A- 5 569 756**

- **VOS, J. N. ET AL: "HPLC detection of sulfated carbohydrates with a new detection method based on measurement of optical activity by a polarized laser (Chiramonitor)" JOURNAL OF CARBOHYDRATE CHEMISTRY , 9(4), 501-5 CODEN: JCACDM; ISSN: 0732-8303, 1990, XP008034866**
- **DAMM, JAN B. L. ET AL: "Indirect UV detection as a non-selective detection method in the qualitative and quantitative analysis of heparin fragments by high-performance capillary electrophoresis" JOURNAL OF CHROMATOGRAPHY, A , 678(1), 151-65 CODEN: JCRAEY; ISSN: 0021-9673, 1994, XP001173074**
- **KELLENBACH, E. ET AL: "Using pulse field gradient NMR-based diffusion experiments to identify signals of low-molecular-weight impurities" ORGANIC PROCESS RESEARCH & DEVELOPMENT , 3(2), 141-144 CODEN: OPRDFK; ISSN: 1083-6160, 1999, XP001202114**
- **KUBERAN, BALAGURUNATHAN ET AL: "Analysis of heparan sulfate oligosaccharides with ion pair-reverse phase capillary high performance liquid chromatography-microelectrospray ionization time-of-flight mass spectrometry" JOURNAL OF THE AMERICAN CHEMICAL SOCIETY , 124(29), 8707-8718 CODEN: JACSAT; ISSN: 0002-7863, 2002, XP001202115**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **PETITOU, MAURICE ET AL: "Synthesis of heparin fragments: a methyl .alpha.-pentaoside with high affinity for antithrombin III" CARBOHYDRATE RESEARCH , 167, 67-75 CODEN: CRBRAT; ISSN: 0008-6215, 1987, XP001202113**
- **DAMM, JAN B. L. ET AL: "Separation of natural and synthetic heparin fragments by high-performance capillary electrophoresis" JOURNAL OF CHROMATOGRAPHY , 608(1-2), 297-309 CODEN: JOCRAM; ISSN: 0021-9673, 1992, XP001194807**
- **LIU, FENGQI ET AL: "SR-90107 Sanofi-Synthelabo" IDRUGS , 3(9), 1088-1099 CODEN: IDRUFN; ISSN: 1369-7056, 2000, XP008034864**
- **PAOLUCCI FRANCIS ET AL: "Fondaparinux sodium mechanism of action: Identification of specific binding to purified and human plasma-derived proteins" CLINICAL PHARMACOKINETICS, vol. 41, no. Supplement 2, 2002, pages 11-18, XP008034837 ISSN: 0312-5963**
- **DATABASE WPI Section Ch, Week 198823 Derwent Publications Ltd., London, GB; Class B04, AN 1988-158175 XP002295381 & JP 63 098395 A (KATAKURA CHIKKARIN CO LTD) 28 avril 1988 (1988-04-28)**

## Description

La présente invention concerne un procédé de préparation d'une composition de fondaparinux sodique de haute pureté.

Le fondaparinux sodique ou méthyl O-2-déoxy-6-O-sulfo-2-(sulfoamino)-α-D-glucopyranosyl-(1→4 )-O-β-D-glucopyranuronosyl-(1→4)-O-2-déoxy-3,6-di-O-sulfo-2-(sulfoamino)-α-D-glucopyranosyl-(1→4)-2-O-sulfo-α-D-glucopyranosyl-(1→4)-O-2-O-sulfo-L-idopyranurosyl-(1→4)-2-déoxy-6-O-sulfo-2-(sulfoamino)-α-D-glucopyranoside, decakis sel de sodique, composé de formule I

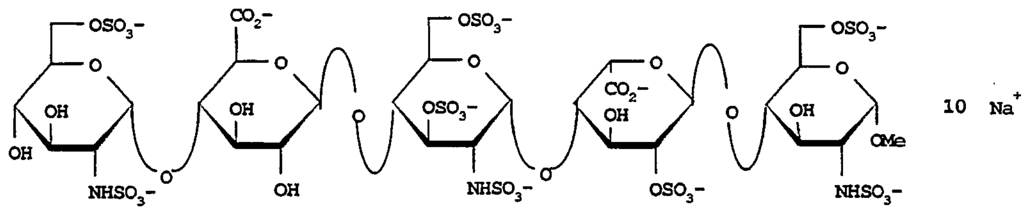

I

est un composé ayant une activité anti facteur Xa (anti Xa) très puissante et des propriétés antithrombotiques très intéressantes. Ce composé qui a une masse moléculaire de 1728 trouve son application dans le traitement et la prévention des maladies thromboemboliques et il est le principe actif de la spécialité Arixtra® qui est administrée par voie sous cutanée.

Ce composé est obtenu selon le procédé décrit dans les brevets EP 084 999 et US 4 818 816. Le fondaparinux sodique est issu d'une synthèse chimique comportant plus de 50 étapes. Ce procédé permet d'obtenir du fondaparinux sodique brut qui est un mélange constitué du fondaparinux sodique et d'autres oligosaccharides apparentés. La teneur de ce mélange en fondaparinux sodique, évalué par chromatographie liquide haute performance (CLHP) d'échange d'anions est d'environ 70%.

Plusieurs étapes de purification par chromatographie sur colonne et par précipitation sont nécessaires pour obtenir du fondaparinux sodique ayant une pureté qui ne dépasse pas 96,0%.

Par ailleurs, le grand nombre d'étapes de synthèse rend la standardisation des lots industriels très difficile.

Etant donné la complexité de la structure du fondaparinux sodique et de ses intermédiaires de synthèse, de nombreuses impuretés peuvent se former au cours de la synthèse. En plus, la moindre variation des conditions opératoires a comme résultat l'obtention des lots de fondaparinux sodique brut contenant des produits apparentés dans des quantités importantes. Ces produits apparentés, qui n'ont pas une activité anti Xa ou qui ont une activité très modeste, ont une structure chimique et des caractéristiques physico-chimiques très proches du fondaparinux sodique et ne peuvent pas être éliminés de manière satisfaisante par des méthodes de purification indiquées ci-dessus. Par ailleurs, il a été observé que certains de ces produits sont facilement dégradables, lorsqu'ils sont soumis à une stérilisation par des méthodes telles que l'autoclavage, et produisent ainsi des impuretés additionnelles.

Le fondaparinux sodique, principe actif d'une spécialité pharmaceutique, doit répondre à certains critères et normes de qualité et être en particulier de la plus haute pureté possible. De ce fait, les lots industriels qui contiennent des produits apparentés en des quantités importantes ne sont pas utilisables pour la préparation des spécialités pharmaceutiques. Ainsi il est important de disposer de compositions de fondaparinux sodique de haute pureté et notamment des quantités industrielles de telles compositions ainsi que d'un procédé permettant de les obtenir.

De manière surprenante, il a été maintenant trouvé qu'en soumettant le fondaparinux sodique brut à au moins une étape de purification sur charbon actif et ensuite à une ou plusieurs étapes de purification de sucres classiques telles que la chromatographie sur colonne ou la précipitation/cristallisation, il est possible d'obtenir du fondaparinux sodique de haute pureté. Il s'agit des compositions constituées en majorité de fondaparinux sodique et d'autres oligosaccharides tels que penta ou octasaccharides, en particulier des pentasaccharides apparentés, qui contiennent au moins 97% de fondaparinux sodique.

Ces compositions ont un grand intérêt au niveau industriel, en particulier dans le domaine pharmaceutique. Elles répondent à une qualité pharmaceutique et peuvent être utilisées comme principe actif des médicaments.

De manière surprenante il a été également trouvé que le seul passage sur charbon actif permet d'obtenir des compositions de constitution bien déterminée et ceci à partir des lots de fondaparinux sodique brut ayant des teneurs en fondaparinux sodique et des impuretés apparentées très différentes.

Le charbon actif est utilisé depuis longtemps pour éliminer les impuretés des gaz et des liquides. Par exemple, on utilise le charbon actif pour épurer les eaux usées et l'eau potable. On utilise également le charbon actif pour éliminer

des traces d'impuretés de structure très différente par rapport au produit à purifier.

Dans le domaine des dérivés des sucres, le charbon actif a été utilisé pour purifier des glucosides. Par exemple, la demande de brevet FR 2 557 139 décrit un procédé de purification des glucosides bruts en solution, mettant en oeuvre une levure, qui permet la diminution des sucres réducteurs mono ou oligosaccharides. Selon ce procédé, le charbon actif est utilisé dans les derniers stades pour décolorer et désodoriser la solution aqueuse du glucoside exempt des oligosaccharides réducteurs.

Par ailleurs, la demande de brevet FR 2 732 024 décrit un procédé de purification de cyclodextrines chimiquement modifiées utilisant du charbon actif dans une phase liquide. Selon ce procédé seul le charbon actif de provenance spécifique, notamment celui obtenu à partir de houille et d'enveloppes de noix de coco permet d'éliminer les impuretés organiques produites lors de la modification chimique des cyclodextrines ainsi que les solvants réactionnels résiduels.

Rares sont les procédés connus mettant en oeuvre le charbon actif qui permettent de purifier des substances en éliminant de grandes quantités d'impuretés ayant une structure similaire à la substance à purifier. On connaît par exemple, la demande de brevet WO 01/16079 qui décrit un procédé de purification de l'acide 2,6-naphtalène carboxylique qui met en oeuvre du charbon actif et un matériel spécifique. Ce procédé permet d'éliminer les impuretés de synthèse, notamment les acides 1 et 2-naphtanoiques. Selon ce procédé, on filtre une solution du produit à purifier sur des microbilles d'absorbant qui contiennent du charbon actif.

La présente invention concerne un procédé de préparation des compositions du fondaparinux sodique de haute pureté y compris celles qui contiennent un petit pourcentage d'impuretés qui sont des oligosaccharides apparentés.

L'analyse par chromatographie liquide haute performance (CLHP) d'échange d'anions et détection par UV à λ=210 mn du fondaparinux sodique purifié, obtenu selon le procédé décrit dans les brevets EP 084 999 et US 4 818 816, a permis d'identifier un certain nombre d'impuretés qui sont des oligosaccharides apparentés. Les impuretés majeures identifiées sont les suivantes :

<u>**Impureté A**</u> (Temps de rétention relatif par rapport au temps de rétention du fondaparinux sodique (Trr) = 0,8)

Méthyl (2-amino-2-déoxy-6-*O*-sodium sulfonato-α-D-glucopyranosyl)-(1→4)-(sodium β-D-glucopyranosyluronate)-(1→4)-(2-déoxy-2-sodium sulfoamino-3,6-di-*O*-sodium sulfonato-α-D-gluocopyranosyl )-(1→4)-(sodium 2-*O*-sodium sulfonato-α-L-idopyranosyluronate)-2-déoxy-2-sodium sulfoamino-6-*O*-sodium sulfonato-α-D-glucopyranoside, composé de formule II :

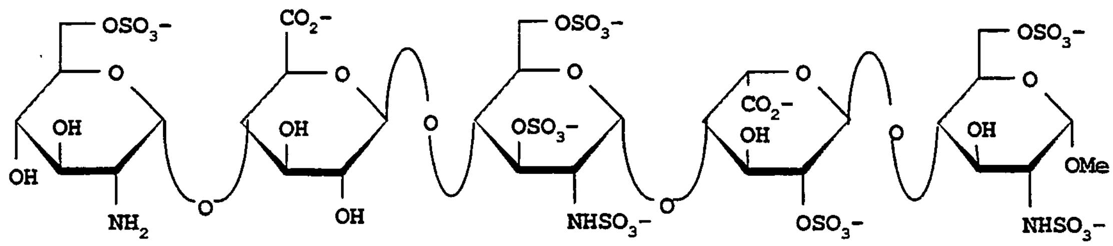

II

<u>**Impureté B**</u> (Trr = 0,93)

Il s'agit d'un mélange constitué, en majorité, de l'impureté méthyl (2-déoxy-2-sodium sulfoamino-6-*O*-sodium sulfonato-α-D-glucopyranosyl)-(1→4)-(sodium β-D-glucopyranosyluronate)-(1→4)-(2-déoxy-2-sodium sulfoamino-3,6-di-*O*-sodium sulfonato-α-D-gluocopyranosyl)-(1→4)-(sodium 2,3-di-*O*-sodium sulfonato-α-L-idopyranosyluronate)-2-dé0oxy-2-sodium sulfoamino-6-*O*-sodium sulfonato-α-D-glucopyranoside, composé de formule III:

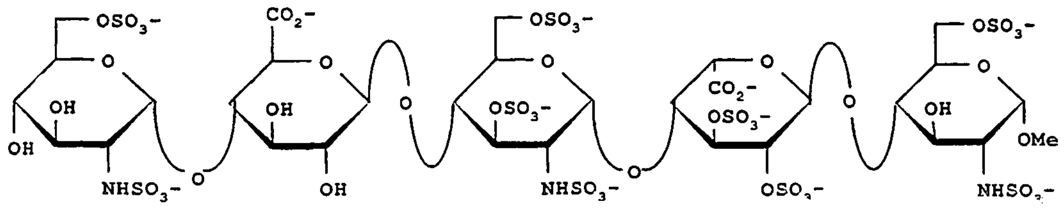

III

et d'impureté méthyl (2-déoxy-2-formylamino-6-*O*-sodium sulfonato-α-D-glucopyranosyl)-(1→4)-(sodium β-D-glucopyranosyluronate)-(→4)-(2-déoxy-2-sodium sulfoamino-3,6-di-*O*-sodium sulfonato-α-D-gluocopyranosyl)-(1→4)-(sodium 2-*O*-sodium sulfonato-α-L-idopyranosyluronate)-2-déxy-2-sodium sulfoamino-6-*O*-sodium sulfonato-α-D-glucopyranoside, composé de formule IV:

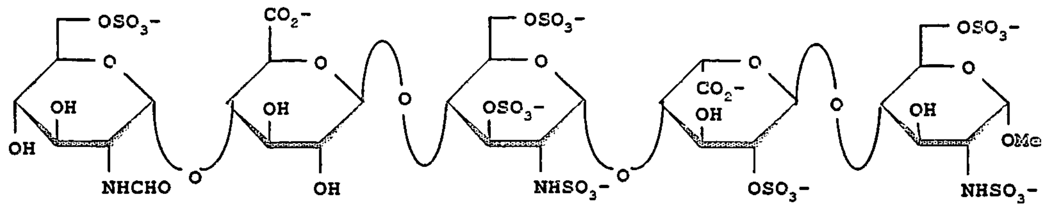

IV

cette dernière, se trouvant dans le mélange à des quantités moindres ou étant souvent absente.

**<u>Impureté C</u>** (Trr = 1,2)

Il s'agit d'un mélange constitué en majorité de méthyl (2-déoxy-2-sodium sulfoamino-6-*O*-sodium sulfonato-α-D-glucopyranosyl)-(1→4)-(sodium 2-*O*-cyclohexylméthyl-β-D-glucopyranosyluronate)-(1→4)-(2-deoxy-2-sodium sulfoamino-3,6-di-*O*-sodium sulfonato-α-D-gluocopyranosyl)-(1→4)-(sodium 2-*O*-sodium sulfonato-α-L-idopyranosyluronate)-2-déoxy-2-sodium sulfoamino-6-*O*-sodium sulfonato-α-D-glucopyranoside, composé de formule V :

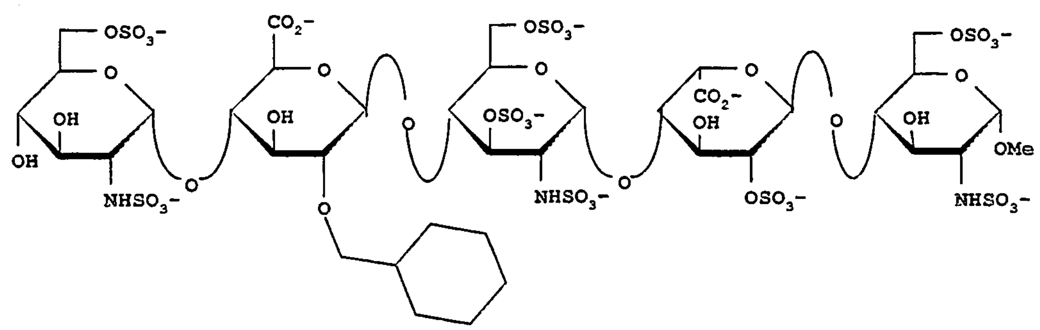

V

**Impureté D)** (Trr =1,3).

Il s'agit du méthyl (sodium 4-*O*-{sodium 7-hydroxy-2-oxo-6-[(sulfonatooxy) méthyl]hexahydro-4-*H*-pyrano [3,4-*d*][1,3]oxazol-4-yl}-β-D-glucopyranosyluronate)-(1→4)-(2-déoxy-2-sodium sulfoamino-3,6-di-*O*-sodium sulfonato-α-D-gluocopyranosyl)-(1→4)-(sodium 2-*O*-sodium sulfonato-α-L-idopyranosyluronate)-2-déoxy-2-sodium sulfoamino-6-*O*-sodium sulfonato-α-D-glucopyranoside, composé de formule VI :

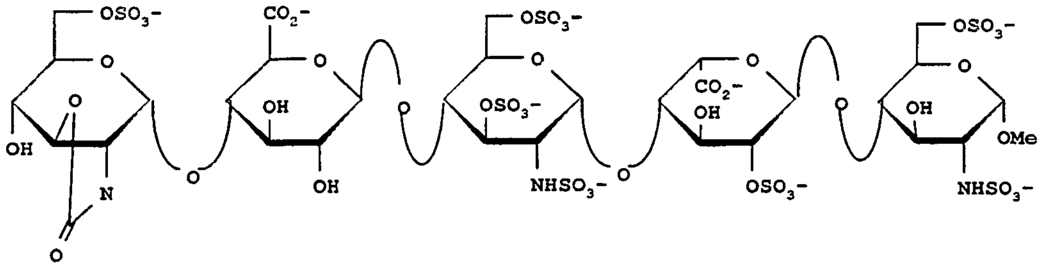

VI

**Impureté E** (Trr =1,4).

Il s'agit du méthyl (3,4-di-*O*-[(2-déoxy-2-sodium sulfoamino-6-*O*-sodium sulfonato-α-D-glucopyranosyl)-(1→)-(sodium β-D-glucopyranosyluronate)-(1→4) -2-déoxy-2-sodium sulfoamino-3,6-di-*O*-sodium sulfonato-α-D-gluocopyranosyl]-2-*O*-sodium sulfonato-a-L-idopyranosyluronate)-2-déoxy-2-sodium sulfoamino-6-*O*-sodium sulfonato-α-D-glucopyranoside, composé de formule VII :

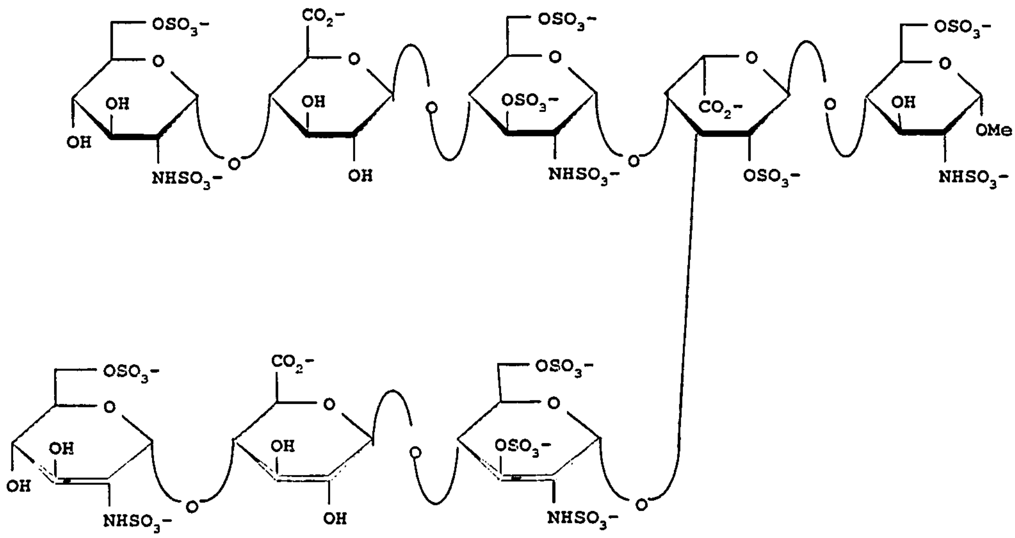

VII

**Impureté F** (Trr = 1,5).

Il s'agit du méthyl (2-déoxy-2-sodium sulfoamino-6-*O*-sodium sulfonato-α-D-glucopyranosyl)-(1→4)-(sodium β-D-glucopyranosyluronate)-(1→4)-(2-déoxy-2-sodium sulfoamino-3,6-di-*O*-sodium sulfonato-α-D-gluocopyranosyl)-(1→4)-(sodium 2-*O*-sodium sulfonato-α-L-idopyranosyluronate)-2-benzamido-2-déoxy-6-*O*-sodium sulfonato-α-D-glucopyranoside, composé de formule VIII :

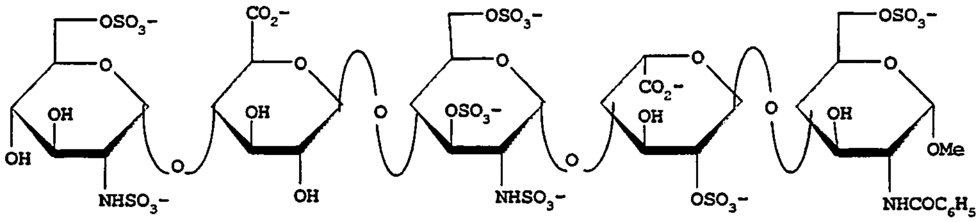

VIII

**Impureté G** (Trr = 1,58).

Il s'agit du méthyl (2-déoxy-2-sodium sulfoamino-6-*O*-sodium sulfonato-α-D-glucopyranosyl)-(1→4)-(sodium 3-*O*-cyclohexylméthyl-β-D-glucopyranosyluronate)-(1→4)-(2-deoxy-2-sodium sulfoamino-3,6-di-*O*-sodium sulfonato-α-D-gluocopyranosyl)-(1→4)-(sodium 2-*O*-sodium sulfonato-α-L-idopyranosyluronate)-2-déoxy-2-sodium sulfoamino-6-*O*-sulfonato-α-D-glucopyranoside, composé de formule IX :

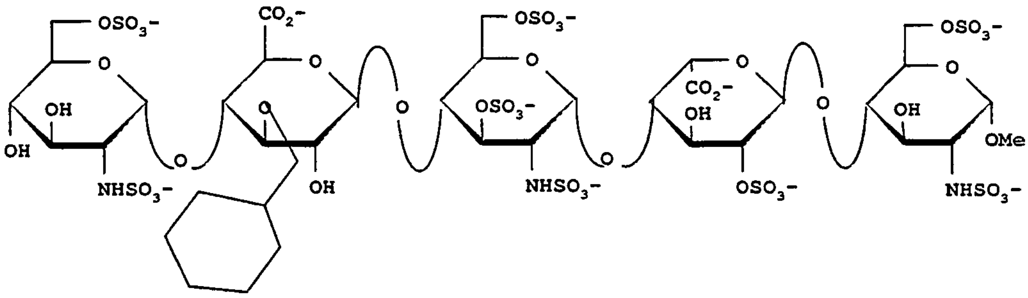

IX

**Impureté H** (Trr= 1,60 )

Il s'agit du méthyl (2-déoxy-4-*O*-{disodium 3,4,8-trihydroxy-11-oxo-9-(sulfonatooxy)-7-[(sulfonatooxy)méthyl] decahydro-2*H*,5a*H*-dipyrano[2,3-b:2,3-*f*] [1,4]oxazepin-2-yl}-2-sodium sulfoamino-3,6-di-*O*-sodium sulfonato-α-D-gluocopyranosyl)-(1→4)-(sodium 2-*O*-sodium sulfonato-α-L-idopyranosyluronate)-2-déoxy-2-sodium sulfoamino-6-*O*-sulfonato-α-D-glucopyranoside, composé de formule X :

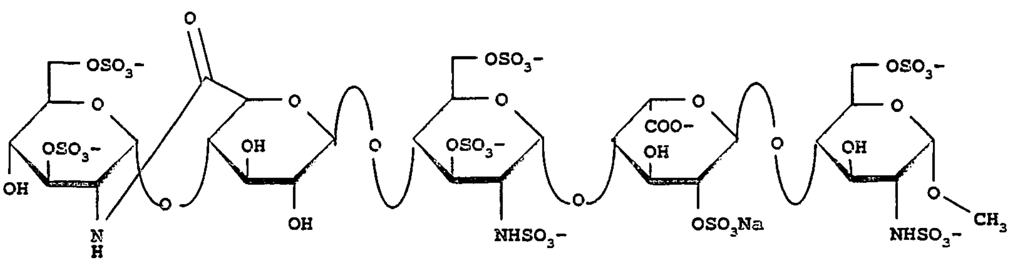

X

Les impuretés indiquées ci-dessus ont été identifiées par spectrométrie de masse et /ou spectrométrie RMN.
Le coefficient d'extinction pour ces composés n'étant pas évalué avec exactitude, la quantité de ces composés dans le fondaparinux sodique brut ou dans les compositions du fondaparinux sodium selon l'invention est exprimée en

pourcentage (%) selon la formule suivante :

$$[A_i / A_{total}] \times 100$$

où

$A_i$: aire du pic correspondant à la substance apparentée « i » observée sur le chromatogramme obtenu avec la solution à analyser.

$A_{total}$ : somme des aires des pics observés dans la solution d'essai.

La présente invention concerné un procédé de préparation d'une composition de fondaparinux sodique de haute pureté, qui contient au moins 97%, de préférence au moins 98% de fondaparinux sodique, le reste des composants étant des oligosaccharides apparentés, étant entendu que la quantité de fondaparinux sodique dans ces compositions ne correspond jamais à 100%. Parmi ces compositions on préfère les compositions pour lesquelles la quantité de l'impureté B est au plus de 0,8% et en particulier parmi ces dernières compositions celles pour lesquelles la quantité de l'impureté D est au plus de 1,0%, de préférence de 0,5%.

La présente invention concerne plus particulièrement un procédé de préparation d'une composition de fondaparinux sodique de haute pureté, qui contient au moins 98% de fondaparinux sodique, le reste des composants étant des oligosaccharides apparentés, étant entendu que la quantité de fondaparinux sodique dans ces compositions ne correspond jamais à 100%, la quantité de l'impureté B étant au plus de 0,8%, la quantité de l'impureté C étant au plus de 1,0% et la quantité de l'impureté D étant au plus de 0,5%. Parmi ces compositions on préfère les composition pour lesquelles la quantité de l'impureté B est au plus de 0,8%, la quantité de l'impureté C est au plus de 0,6% et la quantité de chacune des impuretés oligosaccharides apparentés, autres que les impuretés B et C, est au plus de 0,3%.

La présente invention concerne également plus particulièrement un procédé de préparation d'une composition de fondaparinux sodique de haute pureté, qui contient au moins 98% de fondaparinux sodique, le reste des composants étant des oligosaccharides apparentés, étant entendu que la quantité de fondaparinux sodique dans ces compositions ne correspond jamais à 100%, la quantité de l'impureté B étant au plus de 0,8%, la quantité de l'impureté D étant au plus de 0,5%, et la quantité de chacune d'impuretés oligosaccharides apparentés autres que les impuretés B et D étant au plus de 0,3%. Parmi ces compositions, on préfère les compositions pour lesquelles la quantité de l'impureté D est au plus de 0,3% et en particulier on préfère les compositions pour lesquelles la quantité de l'impureté B est au plus de 0,5% et la quantité de l'impureté D est au plus de 0,3%.

Toutes ces compositions sont de préférence préparées à des quantités industrielles, plus précisément à des quantités de 5 gr à 8.000 gr, par exemple de 10gr, de 100gr, 500gr, 800gr 1.000 gr, 2.000gr, ou 5.000gr et peuvent être obtenue à partir du fondaparinux sodique brut.

Comme indiqué précédemment, le fondaparinux sodique est issu d'une synthèse chimique d'oligosaccharides, comportant plus de 50 étapes. Ce procédé permet d'obtenir du fondaparinux sodique brut qui présente une pureté par CLHP ionique d'environ 70%.

Pour obtenir le procédé de préparation des compositions de la présente invention, on soumet le fondaparinux brut, à l'action du charbon actif. Cette étape, de manière surprenante, permet de réduire de manière conséquente le taux d'impuretés présentes et notamment l'impureté D, composé de structure proche de fondaparinux sodique, mais qui se dégrade très facilement lors des différentes étapes qui permettent la stérilisation des spécialités pharmaceutiques ayant comme principe actif du fondaparinux sodique.

Les compositions ainsi obtenues sont ensuite purifiées avec des méthodes classiques, utilisées jusqu'à présent pour les oligosaccharides, à savoir purification sur colonne de chromatographie et précipitation par l'éthanol. On obtient ainsi des compositions de fondaparinux sodique de haute pureté, ayant une teneur en ce principe actif d'au moins 97%.

La présente invention concerne également un procédé de purification du fondaparinux sodique qui comprend au moins une étape de purification sur le charbon actif.

Le charbon actif utilisé pour obtenir les compositions selon l'invention peut être produit à partir de différents composés tels que végétaux (bois, tourbe, coque de noix de coco ou grain de café), minéraux (charbon, houille ou hydrocarbures) ou animaux (sang, os, etc...).

Ces charbons dits charbons actifs sont activés soit par action de la vapeur, soit par action chimique. La porosité qui en résulte détermine alors les propriétés d'absorption du charbon et lui confère son efficacité. Selon la présente invention, on utilise de préférence du charbon d'origine végétale activé par la vapeur. On préfère le charbon végétal de type Norit A Supra Euro® produit par Norit.

On préfère également du charbon actif de type 3S® fourni par CECA de type CPL® fourni par CECA, de type GAC 1240® fourni par NORIT ou de type 4SC fourni par CECA.

La purification sur charbon actif peut être effectuée sur le fondaparinux sodique brut en solution aqueuse, avant précipitation par l'éthanol ou après précipitation par l'éthanol. De préférence, la purification sur charbon actif est effectuée après avoir procédé à une première purification par précipitation à l'aide de l'éthanol.

Le traitement par le charbon actif peut être effectué soit en système batch agité avec du charbon pulvérulent, soit en système dynamique avec du charbon fixé sur les plaques filtrantes.

On préfère le traitement en système dynamique.

Le ratio du charbon actif par rapport au fondaparinux sodique se situe entre 50% et 150%, de préférence entre 80% et 125%, plus particulièrement à 100%.

Dans les deux cas de figure à savoir traitement par le charbon actif ou en système dynamique sur filtre, la purification est réalisée à une température comprise entre 5°C et 50°C, avantageusement entre 15°C et 35°C et de préférence à température ambiante.

Le pH de la solution du fondaparinux brut à purifier est ajusté, si nécessaire, entre 5 et 10, de préférence entre 7 et 9.

Le filtrat primaire ainsi que les solutions de lavages sont réunis et filtrés sur membrane de porosité inférieure à 1 µm, avantageusement à 0,22µm.

Les exemples suivants illustrent l'invention :

**<u>Détermination du titre du fondaparinux sodique dans les compositions</u>**

La détermination du titre du fondaparinux sodique est effectuée par CLHP ionique et détection par UV à λ=210 nm.

Pour la chromatographie liquide haute performance on utilise a) une colonne échangeuse d'anions de 250 mm de longueur et de 4 mm de diamètre interne remplie d'une matrice polymérique sur laquelle sont greffées des microperles de latex portant des groupements fonctionnels ammonium quaternaires (Dionex Carbopac® réf 035391 ou équivalent) et b) une précolonne échangeuse d'anions de 50 mm de longueur et de 4 mm de diamètre interne remplie avec la même phase stationnaire que la colonne analytique (Dionex Carbopac® réf 043096 ou équivalent). La température des colonnes est maintenue à 30°C durant l'analyse.

Comme phase mobile on utilise un mélange des phases mobiles A (150 µl d'une solution de diméthylsulfoxide diluée au 1/10$^{ème}$ à 1000 ml $H_2O$) et B (dissoudre 117g de NaCl dans 1000 ml $H_2O$)

Le débit de la phase mobile est ajusté à 1 ml /min et on utilise le gradient linéaire suivant :

| Temps (min) | Phase mobile A % | Phase mobile B % |
|---|---|---|
| 0 | 50 | 50 |
| 5 | 50 | 50 |
| 25 | 10 | 90 |
| 30 | 10 | 90 |
| 35 | 50 | 50 |
| 50 | 50 | 50 |

La conformité du système est évaluée avec une solution de référence contenant les impuretés majeures. Un chromatogramme type est donné à la figure 1.

Le pic de l'impureté B (Trr = 0,93) et le pic éluant juste après celui-ci doivent apparaître comme deux pics distincts.

Le pic de l'impureté D et le pic de Trr= 1,29 doivent apparaître comme 2 pics distincts.

Le pic de l'impureté G (Trr = 1,58) et le pic de l'impureté H (Trr= 1,60) doivent apparaître comme 2 pics distincts.

Comme solution de référence (SR) on utilise une solution d'une composition de fondaparinux sodique, en teneur connue en fondaparinux sodique qui est d'environ 10mg/ml. Comme solution d'essai (S2) on utilise une solution contenant 100mg de la composition à analyser dans 10ml d'$H_2O$.

La concentration de fondaparinux sodique dans la composition à analyser exempte d'eau et de solvants est calculée selon la formule suivante :

$$Cref(a) \times [S(a)/Sref(a)] \times V/m \times 100 \times [100/100 - (E+S)]$$

où

Cref(a) : concentration en fondaparinux sodique (mg /ml) de la solution de référence SR
S(a) et Sref(a) : aires des pics de fondaparinux sodique obtenus respectivement avec la solution s'essai S2 et la solution de référence SR
V : volume de dissolution de la prise d'essai pour préparer la solution d'essai S2 (ml)
m : prise d'essai de la substance à analyser pour préparer la solution d'essai S2
E : teneur en eau de la substance à analyser (%)
S : teneur en solvants de la substance à analyser (%).

La teneur des substances apparentées dans la solution à analyser de la composition à analyser exempte d'eau et de solvants est calculée selon la formule suivante :

$$[A_i / A_{total}] \times 100$$

où

Ai: aire du pic correspondant à la substance apparentée « i » observée sur le chromatogramme obtenu avec la solution d'essai S2,
$A_{total}$: Somme des aires des pics observés dans la solution d'essai S2.

**Exemple 1**

**OBTENTION D'UNE COMPOSITION SELON L'INVENTION**

Etape A : Purification du fondaparinux sodique brut - Traitement sur charbon en batch agité

Pour le traitement en système batch agité, le fondaparinux sodique brut est mis en solution dans de l'eau déminéralisée à raison de 40 à 60g de fondaparinux sodique pur par litre.
Le lot de fondaparinux sodique brut utilisé pour cet essai contient 71,4% de fondaparinux sodique.

Le taux d'impureté A est égal à 1,5%,
le taux d'impureté B est égal à 1,7%,
le taux d'impureté C est égal à 0,7%,
le taux de l'impureté D est égal à 1,6%,
le taux d'impureté F est égal à 4,5%,
le taux d'impureté G est égal à 0,3%,
et le taux d'impureté H est de 0,5%.

Le pH de la solution est ajusté à 7,6.
Le teneur en sels de fondaparinux sodique brut est suffisante pour garantir une conductivité en solution permettant l'action du traitement au charbon. Toutefois, si nécessaire, la conductivité pourra être ajustée par apport de chlorure de sodium en poudre entre 30 et 100mS/cm.
Le noir végétal (Norit A Supra Euro® en poudre) est ajouté à 100%.
Le milieu est agité entre 2 et 4 heures, préférentiellement 3 heures, puis filtré.
Le charbon est alors lavé par une solution eau/chlorure de sodium de conductivité de 50mS/cm.
Ce lavage se fait en deux temps avec des volumes égaux à la moitié du volume de la solution engagée initialement avec re-empattage du charbon et filtration.
Le filtrat primaire ainsi que la solution de lavage sont ensuite réunis et filtrés sur membrane de porosité de 0,22 mm.
La composition ainsi obtenue contient 94% du fondaparinux sodique.

Le taux d'impureté A est inférieur à 0, 1 %,
le taux d'impureté B est égal à 0,6%,
le taux d'impureté C est inférieur à 0,1% ,
le taux de l'impureté D est égal à 0,2%,

le taux d'impureté F est inférieur à 0,1 %,
le taux d'impureté G est inférieur à 0,1%,
et le taux d'impureté H est inférieur à 0,1% .
Rendement : 90%

Etape B : Purification par chromatographie échangeuse d'anions

La composition du fondaparinux sodique obtenue à l'étape précédente est ensuite purifiée par chromatographie échangeuse d'anions sur colonne Sépharose Q Fast Flow. La colonne est équilibrée avec du chlorure de sodium (NaCl) 0,2 M. La composition du fondaparinux sodique est dissoute dans l'eau et la conductivité est ajustée avec de l'eau ou du NaCl de manière à être inférieure à 20 mS/cm.
On dépose le produit à purifier à raison de 15 g de fondaparinux sodique par litre de gel. On rince l'alimentation de la colonne par du NaCl 0,2M, puis la colonne est lavée par une solution de NaCl 0,46 M, ce qui permet d'éliminer les impuretés faiblement chargées.
On procède ensuite à l'élution du fondaparinux sodique à l'aide d'une solution de NaCl 0,8M, puis à la régénération de la colonne et à la désorption des impuretés fortement chargées par une solution de NaCl 2,00M.
On analyse des fractions et on réunit les fractions ayant une pureté supérieure ou égale à 95%.
Les solutions purifiées contenant du fondaparinux sodique sont concentrées pour obtenir des solutions contenant 20 à 70 g/l de fondaparinux sodique. La conductivité est ajustée avec de l'eau ou de NaCl de manière à être de 45 à 90 mS/cm. La solution ainsi obtenue est filtrée sur une membrane de microfiltration, puis mélangée avec de l'éthanol, à un rapport 1:5 V/V.
On obtient ainsi par précipitation une composition du fondaparinux sodique de haute pureté ayant les caractéristiques suivantes :

Teneur en fondaparinux sodique supérieur à 99,8%
Teneur en impureté A inférieure à 0, 1 %
Teneur en impureté B inférieure à 0,2%,
Teneur en impureté C inférieure à 0, 1 %
Teneur en impureté D inférieure à 0, 1 %
Teneur en impuretés F, G et H inférieure à 0,1 %
Rendement cumulé : 90,4%

**Exemple 2**

**PURIFICATION DU FONDAPARINUX SODIQUE BRUT PAR CHROMATOGRAPHIE SUR COLONNE ET PRECIPITATION (SANS PURIFICATION SUR CHARBON).**

Etape A

Le lot du fondaparinux brut utilisé pour essai est celui décrit dans l'exemple 1.
Ce lot a été soumis à une purification par chromatographie sur colonne selon les conditions décrites dans l'exemple 1 étape B.

Etape B

Cette purification a été suivie par une deuxième purification sur colonne Sépharose Q Fast Flow selon les conditions suivantes :
La colonne a été équilibrée avec une solution de NaCl 0,4 M. La composition du fondaparinux sodique à purifier à été diluée dans de l'eau et la conductivité est ajustée avec de l'eau ou du NaCl de manière à être inférieure à 35 mS/cm. La solution de fondaparinux sodique a été déposée à raison de 12 à 15 g de fondaparinux sodique par litre de gel.
La ligne de l'alimentation de la colonne a été rincée par du NaCl 0,4M et ensuite la colonne a été rincée par une solution de NaCl 0,48 M (désorption des impuretés faiblement chargées). Ensuite on a procédé à l'élution du fondaparinux par une solution de NaCl 0,75M puis à la régénération de la colonne et la désorption des impuretés fortement chargées par une solution de NaCl 2,00M.
Les différentes fractions ont été analysées, puis celles ayant une pureté supérieure ou égale à 95% ont été rassemblées.
On a procédé ensuite à une nanofiltration puis à une microfiltration et après à une précipitation comme décrit dans l'exemple 1 étape B.

La composition du fondaparinux sodique ainsi obtenue a les caractéristiques suivantes :

Teneur en fondaparinux sodique 96,0%
Teneur en impureté A inférieure à 0, 1 %
Teneur en impureté B inférieure à 0,2%,
Teneur en impureté C 0,7%
Teneur en impureté D 1,4%
Teneur en impureté F 1,2%
Teneur en impureté G inférieure à 0,2%,
Teneur en impureté H 0,6%
Rendement cumulé 88,5%

**Exemple 3**

**OBTENTION D'UNE COMPOSITION SELON L'INVENTION**

Etape A : Purification du fondaparinux sodique brut - Traitement Dynamique sur charbon activé

Pour le traitement dynamique, la préparation de la solution contenant du fondaparinux sodique brut est identique à celle décrite dans l'exemple 1 étape A. La solution utilisée du fondaparinux sodique a une concentration en fondaparinux sodique de 50g par litre d'eau déminéralisée. Le pH est ajusté entre 5 et 10 et la conductivité de la solution est ajustée entre 30 et 100 mS/cm.
Le charbon activé est dans ce cas immobilisé dans un filtre, à l'aide d'un adjuvant type résine entre deux plaques de cellulose (filtre CUNO). Le débit de percolation au travers du filtre est de 1000 1/h/$m^2$. La solution est recyclée pendant deux heures.
Le lavage est effectué avec une solution de NaCl de conductivité 50 mS/cm, d'un volume correspondant à 80% du volume initial engagé. Trois lavages successifs sont réalisés.
Ensuite, le filtrat primaire ainsi que les solutions de lavages sont réunis et filtrés sur membrane de porosité inférieure à 1μm.
Le lot de fondaparinux sodique brut utilisé pour cet essai contient 73% de fondaparinux sodique.

Le taux d'impureté A est égal à 3,9%,
le taux d'impureté B est égal à 3,0%,
le taux d'impureté C est égal à 0,7%,
le taux de l'impureté D est égal à 0,9%,
le taux d'impureté F est égal à 0,3%,
le taux d'impureté G est égal à 0,4%,
et le taux d'impureté H est de 0,3%.
Taux d'autres oligosaccharides apparentés non identifiés : environ 1%.

Après le traitement sur charbon activé, la composition du fondaparinux sodique est la suivante :

Teneur en fondaparinux sodique 90,7%
Teneur en impureté A intérieure à 0,2%
Teneur en impureté B 2,1%,
Teneur en impureté C inférieure à 0,2%
Teneur en impureté D 0,3%
Teneur en impureté F inférieure à 0, 1 %
Teneur en impureté G inférieure à 0,1%
Teneur en impureté H inférieure à 0,1 %
Rendement : 93,1 %

Etape B : Purification par chromatographie échangeuse d'anions

On procédé ensuite comme décrit dans l'exemple 1 étape B. Après analyse des fractions on réunit les fractions ayant une pureté supérieure à 70%.
A la fin de cette étape on obtient une composition du fondaparinux sodique de haute pureté qui a les caractéristiques suivantes :

Teneur en fondaparinux sodique 99, 1 %
Teneur en impureté A inférieure à 0,1 %
Teneur en impureté B 0,5%,
Teneur en impureté C inférieure à 0, 1 %
Teneur en impureté D inférieure à 0,1 %
Teneur en impureté F inférieure à 0, 1 %
Teneur en impureté G inférieure à 0, 1 %
Teneur en impureté H inférieure à 0, 1 %
Somme d'autres oligosaccharides apparentés non identifiés : environ 0,4%

On procède ensuite à une deuxième purification par chromatographie échangeuse d'anions comme décrit dans l'exemple 2, étape B. A la fin de cette étape on obtient une composition du fondaparinux sodique de haute pureté qui a les caractéristiques suivantes :

Teneur en fondaparinux sodique 99,7%
Teneur en impureté A inférieure à 0,1%
Teneur en impureté B 0,3%,
Teneur en impureté C inférieure à 0,1 %
Teneur en impureté D inférieure à 0,1 %
Teneur en impureté F inférieure à 0, 1 %
Teneur en impureté G inférieure à 0,1%
Teneur en impureté H inférieure à 0,1%
Somme d'autres oligosaccharides apparentés non identifiés : inférieur à 0,2%
Rendement cumulé : 90, 1 %.

**Exemple 4**

**OBTENTION D'UNE COMPOSITION SELON L'INVENTION**

Etape A : Purification du fondaparinux sodique brut - Traitement Dynamique sur charbon activé

Pour le traitement dynamique, la préparation de la solution contenant du fondaparinux sodique brut est identique à celle décrite dans l'exemple 1 étape A. La solution utilisée du fondaparinux sodique de cet exemple a une concentration en fondaparinux sodique de 30 à 35g par litre d'eau pour préparations injectables. Le pH est ajusté à 7,1 et la conductivité de la solution est ajustée entre 20 et 30 mS/cm.
Le charbon activé est dans ce cas immobilisé dans un filtre, à l'aide d'un adjuvant type résine entre deux plaques de cellulose (filtre CUNO). Le débit de percolation au travers du filtre est de 100 à 500 1/h/$m^2$. La solution est recyclée pendant deux heures.
Le lavage est effectué avec une solution de NaCl de conductivité de 20 à 35 mS/cm, d'un volume correspondant à 150% du volume initial engagé. Le lavage est effectué en continu, sans re-circulation sur le filtre.
Ensuite, le filtrat primaire ainsi que la solution de lavage sont réunis et filtrés sur membrane de porosité inférieure à 1μm (0,22μm)
Le lot de fondaparinux sodique brut utilisé pour cet essai contient 73,6% de fondaparinux sodique.

Le taux d'impureté A est égal à 2,7%,
le taux d'impureté B est égal à 3,4%,
le taux d'impureté C est égal à 0,9%,
le taux de l'impureté D est égal à 0,5%,
le taux d'impureté F est égal à 0,4%,
le taux d'impureté G est égal à 0,2%,
et le taux d'impureté H est de 0,3%.
Taux d'autres oligosaccharides apparentés non identifiés : environ 18%.

Après le traitement sur charbon activé, la composition du fondaparinux sodique est la suivante :

Teneur en fondaparinux sodique 89,6%
Teneur en impureté A inférieure à 0,2%
Teneur en impureté B 2,5%,

Teneur en impureté C 0,7%
Teneur en impureté D est inférieure à 0,2%
Teneur en impureté F inférieure à 0, 1 %
Teneur en impureté G inférieure à 0,1 %
Teneur en impureté H inférieure à 0,1 %
Rendement : 97%

Etape B : Purification par chromatographie échangeuse d'anions

On procède ensuite comme décrit dans l'exemple 1 étape B. Après analyse des fractions on réunit les fractions ayant une pureté supérieure à 70%.

A la fin de cette étape on obtient une composition du fondaparinux sodique de haute pureté qui a les caractéristiques suivantes :

Teneur en fondaparinux sodique 97%
Teneur en impureté A inférieure à 0,1 %
Teneur en impureté B 0,9%,
Teneur en impureté C 0,2%
Teneur en impureté D inférieure à 0, 1 %
Teneur en impureté F inférieure à 0,1 %
Teneur en impureté G inférieure à 0, 1 %
Teneur en impureté H inférieure à 0,1%
Somme d'autres oligosaccharides apparentés non identifiés : environ 2%
Rendement cumulé : 94%

On procède ensuite à une deuxième purification par chromatographie échangeuse d'anions comme décrit dans l'exemple 2, étape B. A la fin de cette étape on obtient une composition du fondaparinux sodique de haute pureté qui a les caractéristiques suivantes :

Teneur en fondaparinux sodique 99%
Teneur en impureté A inférieure à 0,1 %
Teneur en impureté B 0,2%,
Teneur en impureté C inférieure à 0,1 %
Teneur en impureté D inférieure à 0, 1 %
Teneur en impureté F inférieure à 0,1%
Teneur en impureté G inférieure à 0, 1 %
Teneur en impureté H inférieure à 0,1 %
Somme d'autres oligosaccharides apparentés non identifiés : environ 0,8%
Rendement cumulé : 87,7%.

Les solutions purifiées contenant du fondaparinux sodique sont concentrées pour obtenir des solutions contenant 20 à 70 g/l de fondaparinux sodique. La conductivité est ajustée avec de l'eau ou du NaCl de manière à être de 45 à 90 mS/cm. La solution ainsi obtenue est filtrée sur une membrane de microfiltration, puis mélangée avec de l'éthanol, à un rapport 1:5 V/V.

On obtient ainsi par précipitation une composition du fondaparinux sodique de haute pureté ayant les caractéristiques suivantes :

Teneur en fondaparinux sodique supérieure à 99,1 %
Teneur en impureté A inférieure à 0, 1 %
Teneur en impureté B inférieure à 0,2%,
Teneur en impureté C inférieure à 0,3%
Teneur en impureté D inférieure à 0,1 %
Teneur en impuretés F, G et H inférieure à 0, 1 %
Rendement cumulé : 78%

## Revendications

**1.** Procédé de purification du fondaparinux sodique qui contient au moins 98% de fondaparinux sodique, le reste des composants étant des oligosaccharides apparentés, étant entendu que la quantité de fondaparinux sodique dans ces compositions ne correspond jamais à 100%, la quantité de l'impureté B étant au plus de 0.8%, la quantité de l'impureté C étant au plus de 1.0% et la quantité de l'impureté D étant au plus de 0.5% qui comprend au moins une étape de purification sur le charbon actif où a) l'impureté B s'agit d'un mélange constitué, en majorité, de composé de formule III

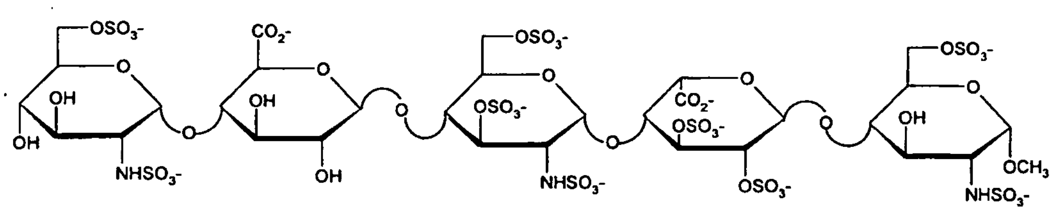

III

et d'impureté composé de formule IV

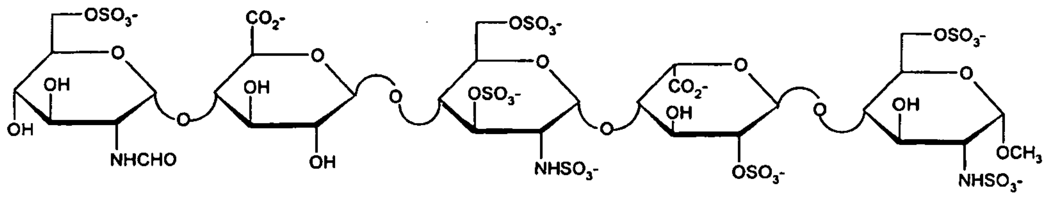

IV

cette dernière, se trouvant dans le mélange à des quantités moindres ou étant souvent absente, b) l'impureté C s'agit d'un mélange constitué en majorité de composé de formule V

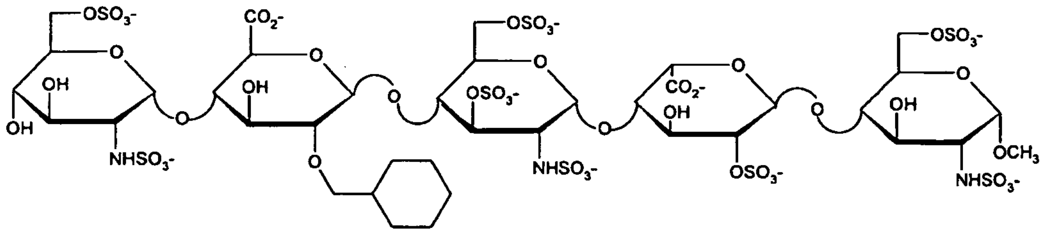

V

et c) l'impureté D s'agit du composé de formule VI

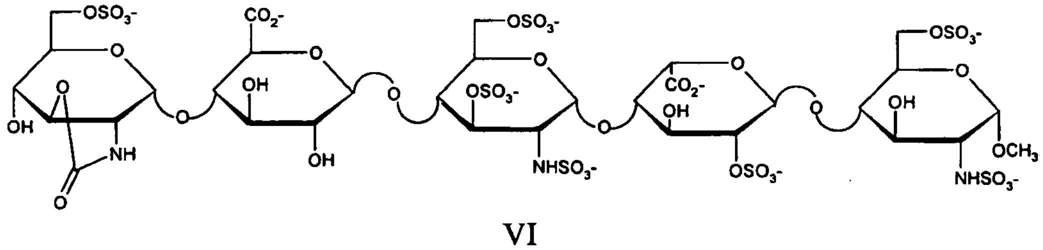

VI

2. Procédé de purification du fondaparinux sodique selon la revendication 1, où le charbon activé est immobilisé dans un filtre, à l'aide d'un adjuvant type résine entre deux plaques de cellulose.

3. Procédé de purification du fondaparinux sodique selon la revendication 1 ou 2 qui met en oeuvre du charbon d'origine végétale activée par la vapeur.

4. Procédé de purification du fondaparinux sodique selon l'une quelconque des revendications 1 à 3 qui est suivi d'au moins une étape de purification par chromatographie sur colonne.

5. Procédé de purification du fondaparinux sodique selon les revendications 1 à 4 qui comprend au moins une étape de précipitation par l'àlcool éthylique.

6. Procédé de purification du fondaparinux sodique qui contient au moins 98% de fondaparinux sodique, le reste des composants étant des oligosaccharides apparentés, étant entendu que la quantité de fondaparinux sodique dans ces compositions ne correspond jamais à 100%, la quantité de l'impureté B est au plus de 0.8%, la quantité de l'impureté C est au plus de 0.6% et la quantité de chacune des impuretés oligosaccharides apparentés, autres que les impuretés B et C, est au plus de 0.3%, qui comprend au moins une étape de purification sur le charbon actif où les impuretés B, C, D sont comme définis dans la revendication 1.

7. Procédé de purification du fondaparinux sodique selon la revendication 6, où le charbon activé est immobilisé dans un filtre, à l'aide d'un adjuvant type résine entre deux plaques de cellulose.

8. Procédé de purification du fondaparinux sodique selon la revendication 6 ou 7 qui met en oeuvre du charbon d'origine végétale activée par la vapeur.

9. Procédé de purification du fondaparinux sodique selon l'une quelconque des revendications 6 à 8 qui est suivi d'au moins une étape de purification par chromatographie sur colonne.

10. Procédé de purification du fondaparinux sodique selon les revendications 6 à 9 qui comprend au moins une étape de précipitation par l'alcool éthylique.

11. Procédé de purification du fondaparinux sodique qui contient au moins 98% de fondaparinux sodique, le reste des composants étant des oligosaccharides apparentés, étant entendu que la quantité de fondaparinux sodique dans ces compositions ne correspond jamais à 100%, la quantité de l'impureté B est au plus de 0.5% et la quantité de l'impureté D est au plus de 0.3% qui comprend au moins une étape de purification sur le charbon actif où les impuretés B, C, D sont comme définis dans la revendication 1.

12. Procédé de purification du fondaparinux sodique selon la revendication 11, où le charbon activé est immobilisé dans un filtre, à l'aide d'un adjuvant type résine entre deux plaques de cellulose.

13. Procédé de purification du fondaparinux sodique selon la revendication 11 ou 12 qui met en oeuvre du charbon d'origine végétale activée par la vapeur.

14. Procédé de purification du fondaparinux sodique selon l'une quelconque des revendications 11 à 13 qui est suivi d'au moins une étape de purification par chromatographie sur colonne.

15. Procédé de purification du fondaparinux sodique selon les revendications 11 à 14 qui comprend au moins une étape de précipitation par l'alcool éthylique.

## Claims

1. A process for purifying fondaparinux sodium which contains at least 98% fondaparinux sodium, the remaining components being related oligosaccharides, wherein the amount of fondaparinux sodium in these compositions never corresponds to 100%, the amount of impurity B is at most 0.8%, the amount of impurity C is at most 1.0% and the amount of impurity D is at most 0.5%, which comprises at least one step of treatment on activated charcoal, wherein a) impurity B is a mixture consisting mainly of a compound of formula III :

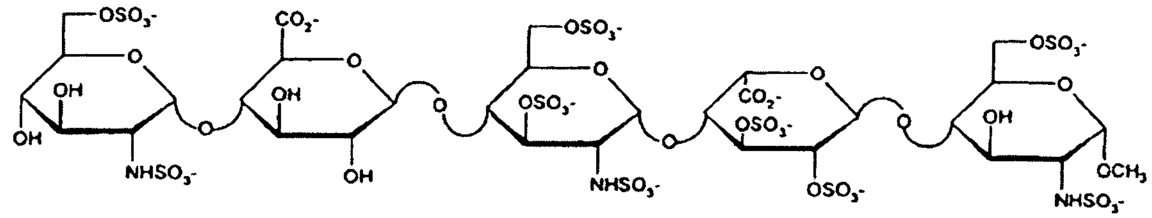

III

and of an impurity compound of formula IV :

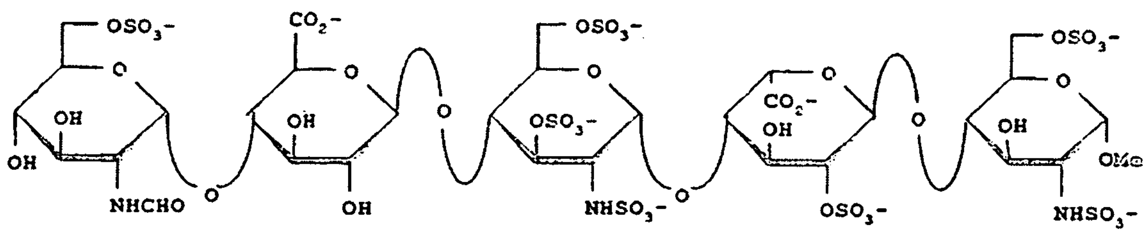

IV

the latter being present in the mixture in lesser amounts, or often being absent, b) impurity C is a mixture consisting mainly of a compound of formula V :

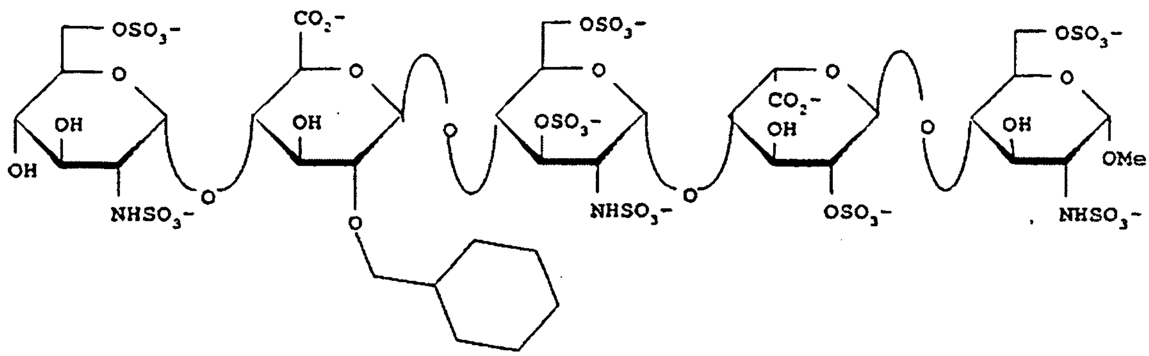

V

and c) impurity D is a compound of formula VI :

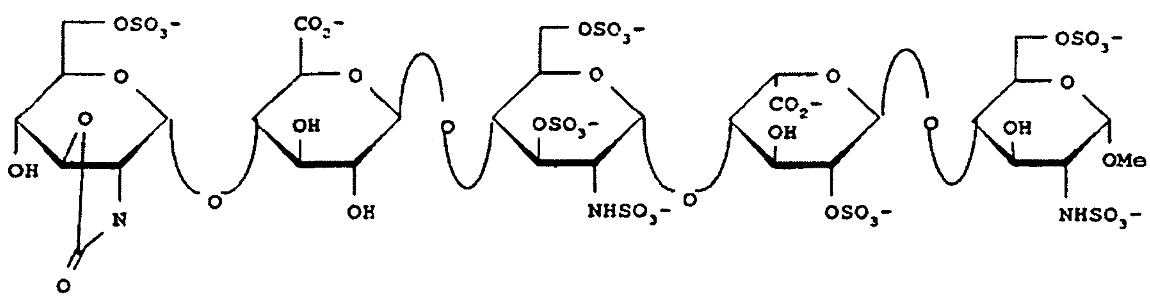

VI

2. The process for purifying fondaparinux sodium according to claim 1, wherein the activated charcoal is immobilized

in a filter, using a resin-type additive, between two cellulose plates.

**3.** The process for purifying fondaparinux sodium according to claim 1 or 2, which uses steam-activated charcoal of vegetal origin.

**4.** The process for purifying fondaparinux sodium according to any of claims 1 to 3, which is followed by at least one step of purification by column chromatography.

**5.** The process for purifying fondaparinux sodium according to any of claims 1 to 4, which comprises at least one step of precipitation from ethyl alcohol.

**6.** A process for purifying fondaparinux sodium which contains at least 98% fondaparinux sodium, the remaining components being related oligosaccharides, wherein the amount of fondaparinux sodium in these compositions never corresponds to 100%, the amount of impurity B is at most 0.8%, the amount of impurity C is at most 0.6% and the amount of each of related oligosaccharide impurities, other than impurities B and C, is at most 0.3%, which comprises at least one step of treatment on activated charcoal, wherein impurities B, C, and D are as defined in claim 1.

**7.** The process for purifying fondaparinux sodium according to claim 6, wherein the activated charcoal is immobilized in a filter, using a resin-type additive, between two cellulose plates.

**8.** The process for purifying fondaparinux sodium according to claim 6 or 7, which uses steam-activated charcoal of vegetal origin.

**9.** The process for purifying fondaparinux sodium according to any of claims 6 to 8, which is followed by at least one step of purification by column chromatography.

**10.** The process for purifying fondaparinux sodium according to any of claims 6 to 9, which comprises at least one step of precipitation from ethyl alcohol.

**11.** A process for purifying fondaparinux sodium which contains at least 98% fondaparinux sodium, the remaining components being related oligosaccharides, wherein the amount of fondaparinux sodium in these compositions never corresponds to 100%, the amount of impurity B is at most 0.5% and the amount of impurity D is at most 0.3%, which comprises at least one step of treatment on activated charcoal, wherein impurities B, C, and D are as defined in claim 1.

**12.** The process for purifying fondaparinux sodium according to claim 11, wherein the activated charcoal is immobilized in a filter, using a resin-type additive, between two cellulose plates.

**13.** The process for purifying fondaparinux sodium according to claim 11 or 12, which uses steam-activated charcoal of vegetal origin.

**14.** The process for purifying fondaparinux sodium according to any of claims 11 to 13, which is followed by at least one step of purification by column chromatography.

**15.** The process for purifying fondaparinux sodium according to any of claims 11 to 14, which comprises at least one step of precipitation from ethyl alcohol.

## Patentansprüche

**1.** Verfahren zur Reinigung von Fondaparinux-Natrium, welches mindestens 98% Fondaparinux-Natrium enthält, wobei die restlichen Bestandteile verwandte Oligosaccharide sind, wobei es sich versteht, dass die Menge an Fondaparinux-Natrium in den Zusammensetzungen nie 100% entspricht, die Menge an Verunreinigung B höchstens 0,8% beträgt, die Menge an Verunreinigung C höchstens 1,0% beträgt, und die Menge an Verunreinigung D höchstens 0,5% beträgt, umfassend mindestens einen Schritt der Reinigung auf Aktivkohle, wobei a) die Verunreinigung B ein Gemisch ist, das hauptsächlich aus einer Verbindung der Formel III

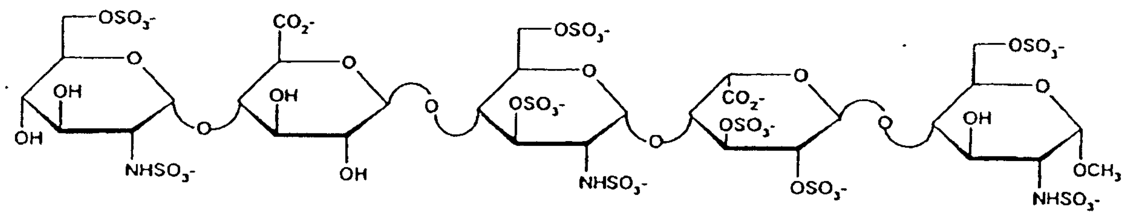

III

und einer Verunreinigungsverbindung der Formel IV besteht

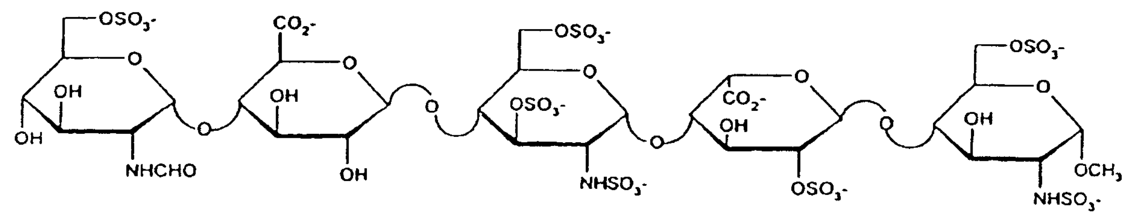

IV

wobei letztere in dem Gemisch in geringfügigen Mengen vorhanden oder oft abwesend ist, b) die Verunreinigung C ein Gemisch ist, das hauptsächlich aus einer Verbindung der Formel V besteht,

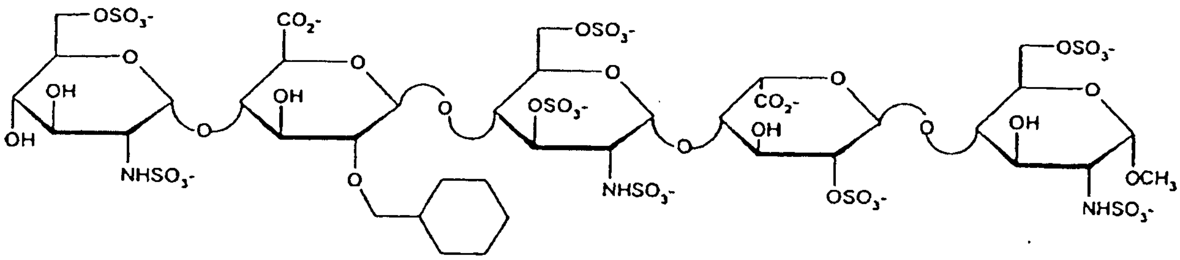

V

und c) die Verunreinigung D eine Verbindung der Formel VI

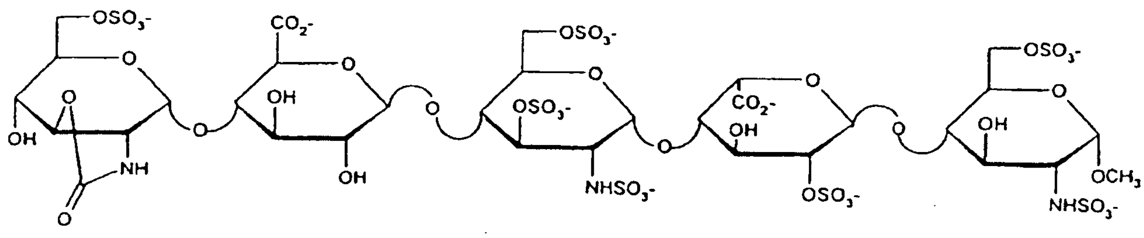

VI

ist.

2. Verfahren zur Reinigung von Fondaparinux-Natrium nach Anspruch 1, wobei die Aktivkohle in einem Filter mit Hilfe eines harzartigen Adjuvans zwischen zwei Celluloseplatten immobilisiert ist.

3. Verfahren zur Reinigung von Fondaparinux-Natrium nach Anspruch 1 oder 2 unter Verwendung von mit Dampf aktivierter Kohle pflanzlichen Ursprungs.

**4.** Verfahren zur Reinigung von Fondaparinux-Natrium nach einem der Ansprüche 1 bis 3, gefolgt von mindestens einem Schritt der Reinigung durch Säulenchromatographie.

**5.** Verfahren zur Reinigung von Fondaparinux-Natrium nach den Ansprüchen 1 bis 4, umfassend mindestens einen Schritt des Fällens mit Ethylalkohol.

**6.** Verfahren zur Reinigung von Fondaparinux-Natrium, welches mindestens 98% Fondaparinux-Natrium enthält, wobei die restlichen Bestandteile verwandte Oligosaccharide sind, wobei es sich versteht, dass die Menge an Fondaparinux-Natrium in den Zusammensetzungen nie 100% entspricht, die Menge an Verunreinigung B höchstens 0,8% beträgt, die Menge an Verunreinigung C höchstens 0,6% beträgt und die Menge an jeglichen, von Verunreinigungen B und C verschiedenen, verwandten Oligosacchariden als Verunreinigungen höchstens 0,3% beträgt, umfassend mindestens einen Schritt der Reinigung auf Aktivkohle, wobei die Verunreinigungen B, C, D wie in Anspruch 1 definiert sind.

**7.** Verfahren zur Reinigung von Fondaparinux-Natrium nach Anspruch 6, wobei die Aktivkohle in einem Filter mit Hilfe eines harzartigen Adjuvans zwischen zwei Celluloseplatten immobilisiert ist.

**8.** Verfahren zur Reinigung von Fondaparinux-Natrium nach Anspruch 6 oder 7 unter Verwendung von mit Dampf aktivierter Kohle pflanzlichen Ursprungs.

**9.** Verfahren zur Reinigung von Fondaparinux-Natrium nach einem der Ansprüche 6 bis 8, gefolgt von mindestens einem Schritt der Reinigung durch Säulenchromatographie.

**10.** Verfahren zur Reinigung von Fondaparinux-Natrium nach den Ansprüchen 6 bis 9, umfassend mindestens einen Schritt des Fällens mit Ethylalkohol.

**11.** Verfahren zur Reinigung von Fondaparinux-Natrium, welches mindestens 98% Fondaparinux-Natrium enthält, wobei die restlichen Bestandteile verwandte Oligosaccharide sind, wobei es sich versteht, dass die Menge an Fondaparinux-Natrium in den Zusammensetzungen nie 100% entspricht, die Menge an Verunreinigung B höchstens 0,5% beträgt und die Menge an Verunreinigung D höchstens 0,3% beträgt, umfassend mindestens einen Schritt der Reinigung auf Aktivkohle, wobei die Verunreinigungen B, C, D wie in Anspruch 1 definiert sind.

**12.** Verfahren zur Reinigung von Fondaparinux-Natrium nach Anspruch 11, wobei die Aktivkohle in einem Filter mit Hilfe eines harzartigen Adjuvans zwischen zwei Celluloseplatten immobilisiert ist.

**13.** Verfahren zur Reinigung von Fondaparinux-Natrium nach Anspruch 11 oder 12 unter Verwendung von mit Dampf aktivierter Kohle pflanzlichen Ursprungs.

**14.** Verfahren zur Reinigung von Fondaparinux-Natrium nach einem der Ansprüche 11 bis 13, gefolgt von mindestens einem Schritt der Reinigung durch Säulenchromatographie.

**15.** Verfahren zur Reinigung von Fondaparinux-Natrium nach den Ansprüchen 11 bis 14, umfassend mindestens einen Schritt des Fällens mit Ethylalkohol.

FIGURE 1

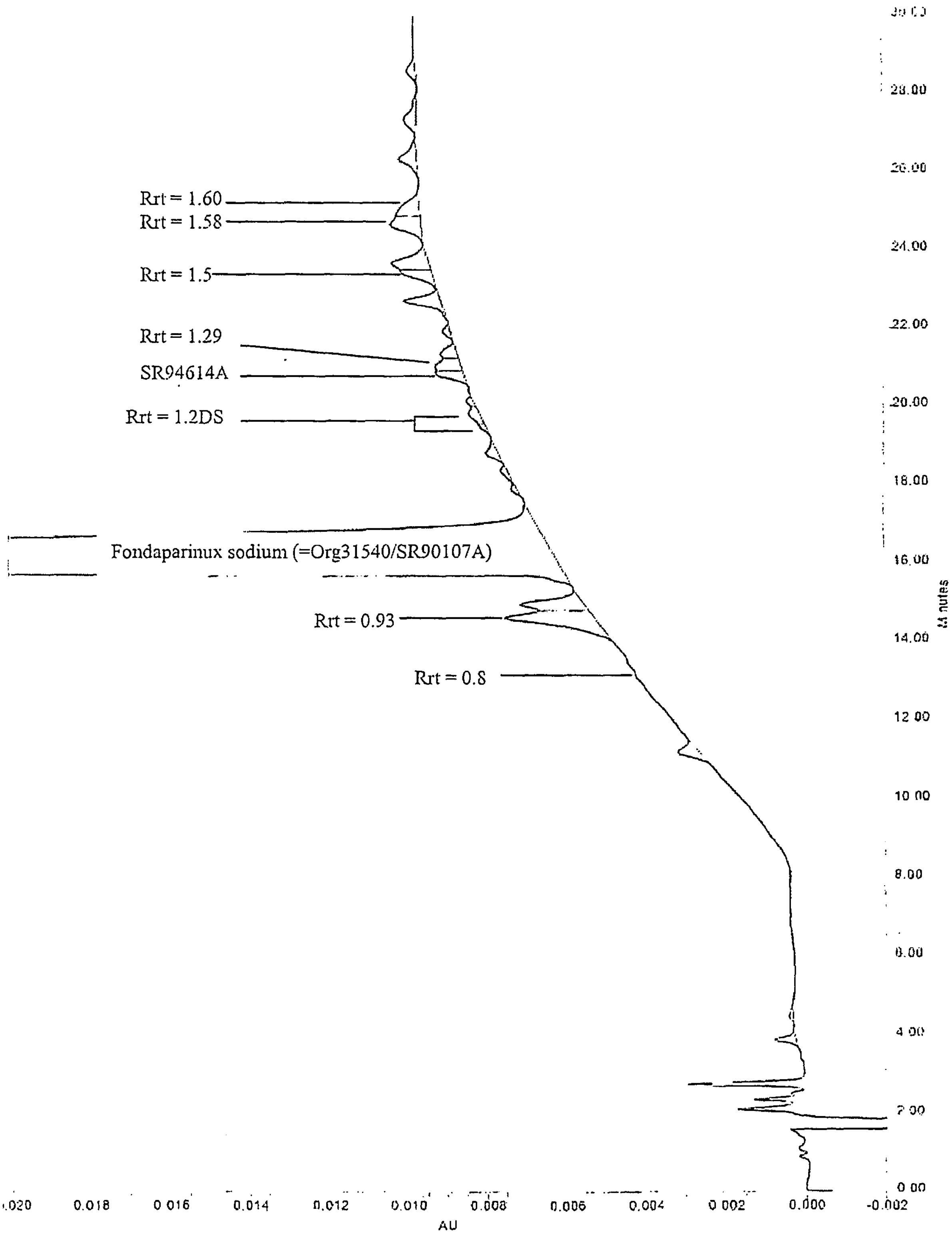
Rrt = 1.60
Rrt = 1.58
Rrt = 1.5
Rrt = 1.29
SR94614A
Rrt = 1.2DS
Fondaparinux sodium (=Org31540/SR90107A)
Rrt = 0.93
Rrt = 0.8
28.00
24.00
22.00
20.00
18.00
16.00
14.00
12.00
10.00
8.00
4.00
0.00
Minutes
0.018
0.014
0.012
0.010
0.008
0.006
0.004
0.002
0.000
-0.002
AU

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 084999 A
- US 4818816 A
- FR 2557139
- FR 2732024
- WO 0116079 A